# EUROPEAN PATENT SPECIFICATION

(11) **EP 1 138 333 B1**
(45) Date of publication and mention of the grant of the patent: **25.02.2004**
(21) Application number: 00966405.3
(22) Date of filing: 11.10.2000
(51) Int. Cl.: A61K 47/26, A61K 9/16, A61K 9/10, A61K 31/431, C07D 499/00, A61K 9/00

(54) **MEDICINAL COMPOSITIONS FOR ORAL USE**
ARZNEIMITTEL ZUR ORALEN VERABREICHUNG
COMPOSITIONS MEDICAMENTEUSES POUR ADMINISTRATION ORALE

(30) Priority: 12.10.1999 JP 29034199
(43) Date of publication of application: 04.10.2001
(73) Proprietor: Daiichi Suntory Pharma Co., Ltd., Tokyo (JP)
(72) Inventor: NOMURA, Masaaki, Ohra-gun, Gunma 370-0708 (JP); KOJIMA, Takumi, Ashikaga-shi, Tochigi 326-0822 (JP); NOZAWA, Shigenori, Adachi-ku, Tokyo 120-0023 (JP)
(74) Representative: HOFFMANN - EITLE
(86) International application number: PCT/JP2000/007045
(87) International publication number: WO 2001/026691

(56) References cited:
- EP-A- 0 171 362
- EP-A- 0 758 651
- EP-A1- 0 444 692
- EP-A1- 0 482 580
- WO-A1-98/03202
- WO-A1-99/36098
- GB-A- 2 124 614
- JP-A- 6 157 312
- JP-A- 7 188 057
- JP-A- 10 045 619
- JP-A- 11 071 285
- JP-A- 59 175 414
- JP-A- 2000 086 520
- US-A- 3 867 523
- US-A- 3 932 615
- US-A- 4 177 254
- US-A- 4 508 723
- US-A- 4 540 689
- US-A- 4 761 408
- US-A- 4 826 832
- US-A- 5 213 806
- US-A- 5 516 531

## Description

### RELATED APPLICATION

The present application claims priority from Japanese Patent Application No. 290341/99 filed on October 12, 1999 the disclosure of which is incorporated herein by reference.

### FIELD OF THE INVENTION

The present invention relates to an oral pharmaceutical composition, which rapidly disintegrates or becomes dissolved or suspended in the mouth during administration or upon addition of water before use.

### PRIOR ART

Oral formulations require compliance with a dosage regimen and thus need to be designed to secure compliance. Especially for children and seniors who have less capacity to swallow, ease of swallowing is an important factor. Oral formulations suitable for children and seniors include oral granular formulations such as granules, powders and fine granules. Oral granular formulations are characterized in that they are smaller than tablets and capsules, which makes them easier to swallow, and also to adjust their dose. Solutions have similar advantages. However, active ingredients supplied in solutions are limited because such ingredients need to be stable in an aqueous,solution. Recently, fast-disintegrating tablets which rapidly disintegrate in the mouth have been proposed. However, it is difficult to adjust the dose in relation to such tablets in spite of their excellent handling properties. Therefore, oral granular formulations are desirable for use in children and seniors to treat various kinds of diseases.

Oral granular formulations are actually administered 1) directly via oral route, optionally with water just following administration, or 2) after being dissolved or suspended in water or the like, or 3) per tubam via a catheter or the like after being dissolved or suspended in water or the like.

In the cases of 1) and 2), rejection by patients of dosing must be avoided especially by children. Such rejection is caused by not only taste and odor but also an sensation to the tongue during administration. That is, any presence of insufficiently disintegrating masses may give rise to oral or guttural discomfort on account of which children are liable to reject a second or subsequent dose. In seniors, insoluble or sparingly soluble particles may enter the gaps between false teeth to cause pain if oral granular formulations poorly disintegrate. This decreases patient compliance and jeopardizes treatment with the proper dosage regimen. In the cases of 2) and 3), rapid dissolution or suspension would be desirable in the field of therapy, and in the case of 3), slow-dissolving or less soluble ingredients in compositions are undesirable since they may cause clogging of tubes.

Thus, while disintegration and dissolution are important properties for oral granular formulations, the design of fast-disintegrating or -dissolving oral granular formulations has not been achieved sufficiently.

Oral granular formulations also suffer from problems with respect handling properties, such as deposition to packing sheets or containers or scattering in the case of powdery formulations having a small particle size. Therefore, appropriately granulated formulations less prone to such problems would be preferred. In addition, oral granular formulations are commonly prepared by adding binders during the granulation process. To granulate mixed powders having low granulation properties, it is necessary to select binders having a high binding power or to use a large amount of binders. This improves granulation, but the resulting granules are hard and generally poor in disintegration or dissolution qualities, and are thus not suitable for oral granular formulations.

### SUMMARY OF THE INVENTION

Thus, the inventors studied carefully the development of a widely applicable technique for use in preparing oral granular formulations; with the resulting formulations having good handling properties, and able to rapidly disintegrate or dissolve, either in the mouth during administration, or when they are dissolved or suspended before use. As a result, the inventors accomplished the present invention on the basis of the finding that oral granular formulations showing rapid disintegration or dissolution in the mouth during administration or when they are dissolved or suspended before use can be prepared by granulation with sucrose with a reduced amount of binders.

### DETAILED DESCRIPTION OF THE INVENTION

Accordingly, the present invention relates to fast-disintegrating oral pharmaceutical compositions granulated with sucrose. According to the present invention, oral granular formulations can be prepared with a reduced amount of binders to provide fast-disintegrating or fast-dissolving oral granular formulations. According to the present invention, it is possible to further improve oral granular formulations, and thuspatient compliance, by incorporating other additives for improving organoleptic properties or stability of active ingredients when the oral granular formulation is required to contain sucrose as a sweetening agent such as a dry syrup.

The fast-disintegrating oral pharmaceutical composition of the present invention can be prepared by granulating a mixture of an active ingredient, sucrose and optionally other additive(s).

The pharmaceutical composition of the present invention is described in detail below.

The amount of sucrose added into the composition of the present invention is not specifically limited so far as it is within a range that allows granulation, but is selected appropriately from a range satisfying pharmaceutically desirable properties, depending on the nature or amount of active ingredients or other ingredients. The amount of sucrose required to be used to obtain good granules is preferably 30% by weight or more of the total composition.

Suitable active ingredients for use in the present invention are not specifically limited so far as they can be administered as oral granular formulations. Examples are drugs for circulatory diseases such as pilsicainide hydrochloride, molsidomine, vinpocetine, propranolol, methyldopa, dipyridamole, furosemide, triamterene, nifedipine, atenolol, spironolactone, metoprolol, pindolol, captopril, isosorbide dinitrate; drugs for central nervous system disorders such as diazepam, idebenone, aspirin, ibuprofen, paracetamol, naproxen, piroxicam, diclofenac, indomethacin, sulindac, lorazepam, nitrazepam, phenytoin, acetaminophen, ethenzamide, ketoprofen; drugs for gastrointestinal disease such as cimetidine, ranitidine, pancreatin, 5-aminosalicylic acid, lansoprazol; drugs for respiratory diseases such as amlexanox, dextromethorphan, theophyline, pseudoephedrine, salbutamol, guaifenesin; antibiotics and chemotherapeutics such as cefalexin, cefaclor, cefradine, amoxicillin, pivampicillin, bacampicillin, dicloxacillin, erythromycin, erythromycin stearate, lincomycin, doxycycline, trimethoprim, sulfamethoxazole and compound 1 below; drugs for metabolic disorders such as serrapeptase, glibenclamide, potassium chloride; vitamin drugs such as vitamin B1, vitamin B2, vitamin B6, vitamin C, fursultiamine. These compounds may be used as salts with alkali metals or alkali earth metals such as sodium, potassium, calcium or magnesium; amino acids such as lysine; ammonium; inorganic acids such as hydrochloric acid or nitric acid; or organic acids such as acetic acid or citric acid; or solvates such as hydrates so far as they are safe such that orally non-toxic and are pharmaceutically acceptable.

When the pharmaceutical composition of the present invention is required to be water-soluble, active ingredients can be selected appropriately from water-soluble compounds. As used herein, the description "water-soluble compounds" includes very soluble, freely soluble or sparingly soluble compounds as defined in the general notices of the 12th edition of the Japanese Pharmacopoeia, i.e., compounds that require less than 100 ml of water to dissolve 1 g or 1 ml of solute.

The pharmaceutical composition of the present invention can be used preferably in solution or syrup when the active ingredient is unstable in water, due to the ability to be use other additives. As a preferred embodiment, the present invention is applied to penem compounds that are typically unstable in water.

Penem compounds are non-natural β-lactam compounds the design of which is based on the concept of fusing the structures of penicillin and cephalosporin (for example, see Woodward, R. B., In Recent Advances in the Chemistry of β-Lactam Antibiotics, Elks, J., Ed., The Chemical Society, London, 1977, Spec. No. 28, pp. 167-180: Japanese Patent Public Disclosure (Kokai) Nos. 207387/86, 162694/88, 222486/85 and 119486/79). They are a novel type of antibiotics having both the wide antibacterial spectrum and high safety of penicillin and cephem antibiotics belonging to β-lactam antibiotics, as well as the potent antibacterial activity and high β-lactamase stability of carbapenem antibiotics.

Among penem compounds, tablets of sodium (+)-(5R, 6S)-6-[(R)-1-hydroxyethyl]-7-oxo-3- [(R)-2-tetrahydrofuryl]-4-thia-1-azabicyclo [3.2.0] hept-2-ene-2-carboxylate 5/2 hydrate (faropenem sodium, hereinafter referred to as compound 1) are currently used as therapeutic agents for infectious diseases. Compound 1 is reported to show potent antibacterial activity against not only methicillin-sensitive Staphylococcus aureus (MSSA), Streptococcus pyrogenes and Streptococcus pneumoniae but also gram-positive bacteria for which conventional β-lactam drugs were not effective such as penicillin-resistant pneumococci (PRSP), oral staphylococci and enterococci and also show a wide antibacterial spectrum covering gram-negative bacteria such as Haemophilus influenzae and anaerobic bacteria such as the genus Bacteroides because of its novel skeleton penem ring (Kagaku Ryoho no Ryoiki (The Field of Chemotherapy), Vol. 13, No. 10, pp. 74-80, 1997). This compound is also reported to have potent antibacterial activity against pathogenic bacteria of periodontis such as Porphyromonas gingivalis (CHEMOTHERAPY, Vol. 42, S-1, pp. 38-50, 1994) as well as potent antibacterial activity against bacterial strains from dental infectious diseases which have recently become increasingly resistant (Journal of Japanese Society of Chemotherapy, Vol. 45, No. 11, pp. 965-971, 1997).

Penem compounds used in the present invention are not specifically limited so far as they have antibacterial activity and safety such as non-immunogenicity and oral non-toxicity and are pharmaceutically acceptable. A specific example is compound 1 above. Compound 1 may be substituted at 3-position by 1,4-dioxane-2-yl, ethylsulfanyl, 3-tetrahydrofurylmethyl, methoxymethyl, ((aminocarbonyl)oxy)methyl, (4R)-pyrrolidine-2-thione-4-ylthio and other groups. These compounds may also be used as salts thereof with alkali metals or alkali earth metals such as sodium, potassium, calcium or magnesium, or amino acids such as lysine, or ammonium, and may also be used as solvates such as hydrates.

When it is desired that the pharmaceutical composition of the present invention be soluble in water, penem compounds as active ingredients can be appropriately selected, taking their water solubility into account.

The amount of an active ingredient in the composition can be determined appropriately depending on the nature of the drug, the disease to be treated or other factors. When compound 1 is used, it is incorporated at about 2-20% by weight as a free anhydride on the basis of the total composition.

The dose of thus obtained formulation containing a penem compound, for example, is typically 50-1500 mg (potency), preferably about 100-1000 mg (potency) daily per adult (60 kg) depending on the disease to be treated, conditions, age and other factors. For children, the dose can be calculated on the basis of body weight.

According to the present invention, it provides a pharmaceutical composition with excellent disintegrating or dissolving properties, and may optionally contain various additives selected to improve pharmaceutically necessary general properties such as stability or ease of handling or conditions necessary to improve compliance such as palatability, odor and color. For example, such additives include (1) excipients such as lactose, starch, microcrystalline cellulose, D-mannitol, glucose and calcium phosphate; (2) disintegrating agents such as starch or sodium carboxymethylcellulose; (3) sweetening agents or corrigents such as D-mannitol, D-sorbitol, aspartame and citric acid; (4) plasticizers such as polyethylene glycol: (5) stabilizers such as polyaminocaroboxylic acid chelating agents such as EDTA and salts thereof, sodium pyrosulfite, 1-ascorbic acid; (6) preservatives such as paraoxybenzoate ester or benzalkonium chloride, as well as colorants and flavoring agents. The nature and amount of these additives can be appropriately determined depending on the desired pharmaceutical characteristics such as stability or taste of active ingredients. When the pharmaceutical composition of the present invention is desired to be soluble in water, additives can be appropriately selected, taking their water solubility into account.

Pharmaceutical compositions of the present invention can optionally further contain binders such as hydroxypropylcellulose or polyvinylpyrrolidone in an amount that does not impair rapid disintegration or dissolution of oral granular formulations. In order to ensure rapid disintegration or dissolution, a binder having a low binding ability is used preferably in an amount of 5% by weight or less, especially 2% by weight or less on the basis of the weight of the compositions.

The oral pharmaceutical composition of the present invention can be prepared by any of the known conventional processes for preparing oral granular formulations. Suitable conventional granulation processes are ones such as extruding granulation, agitating granulation, rolling granulation, fluidized bed granulation, comminuting granulation, crushing granulation, among which fluidized bed granulation is especially preferred because it facilitates preparation of granules characterized by 1) high porosity, 2) low bulk density and 3) good dispersion in water.

According to the present invention, oral granular formulations such as granules, powders and fine granules can be prepared. These formulations can not only be administered orally as granules but also be dissolved or suspended in an aqueous solvent before use. As used herein, formulations for which it is desired to be dissolved or suspended before use means formulations that are shipped in a solid form but dissolved or suspended after opening the package before application. Normally, they are dissolved or suspended immediately before application.

A useful dosage form of oral granular formulations is a dry syrup which is a type of syrup but dissolved or suspended before use. Dry syrup is preferred for the reason that a bitter or unpleasant taste of drugs is masked by a sweetening effect of sugars and various flavoring agents, and that palatability is improved by the use of suitable colorants, giving a pleasant color tone or the like. Dry syrups can be dissolved or suspended before use in not only water but also an aqueous liquid such as juice or milk. Thus, the pharmaceutical composition of the present invention showing rapid disintegration when water is added can be preferably embodied in dry syrups which are required to be easy to take even for seniors and children.

The present invention also includes as preferred embodiments, oral granular formulations similar to dry syrups such as granules, powders and fine granules which contain a substantial amount of sucrose, and thus are suitable for being dissolved or suspended before use.

When oral granular formulations such as granules, powders and fine granules, especially dry syrups are dissolved or suspended before use in hospitals, for example, they are often combined with water and left to stand before being taken by inpatients. Also, at home, dry syrups are mostly taken in divided portions after being combined with water. In the case that suspensions are allowed to stand, insoluble ingredients precipitate thereby having a deleterious effect on the homogeneity of active ingredients, leading to improper dosing.

When insoluble ingredients exist in compositions, an sensation on the tongue may cause children to reject doses. In seniors, insoluble ingredients may enter the gaps between false teeth to cause pain. Such effects decrease patient compliance and jeopardize treatment with the proper dosage regimen. For per tubam administration via a catheter or the like, insoluble ingredients may cause clogging of tubes. Therefore, insoluble ingredients in suspensions should be kept as low as possible; and, hence, the provision of oral granular formulations comprising components which are all homogeneously soluble, is highly desirable.

The pharmaceutical composition of the present invention can provide oral granular formulations which form homogeneously clear solutions when dissolved in water. because sucrose has a very good water solubility. Dry syrups which are homogeneously soluble in water are one of the preferred embodiments of the present invention.

As used herein, the description "dry syrups soluble in water" means those which become clear without leaving any trace of insoluble ingredients when they are combined with an appropriate amount of water. Generally, the amount of water in which dry syrups are dissolved or suspended is determined appropriately taking into account (1) the influence of the concentration on the stability of active ingredients, (2) ease of handling in the field of medication, and (3) ease of consumption by patients. For example, Josamy Dry Syrup (Yamanouchi Pharmaceutical) and Erythrocin Dry Syrup W (Dainippon Pharmaceutical) among commercially available dry syrups are used as suspensions at concentrations of 30, 40 and 100 mg (potency)/mL in accordance with the instructions of the package inserts. The pharmaceutical compositions of the present invention can be used as a dry syrup homogeneously soluble in water within a wide concentration range of active ingredients, specifically at a concentration of 5-200 mg (potency)/mL, for example, 40 mg (potency)/mL for antibiotic formulations.

Preferred examples of a dry syrup include antibiotic formulations. Especially, it would be highly desirable if penem compounds having high safety and potent antibacterial activity as described above could be formulated into a dry syrup which is solid until immediately before use, and which ensures proper compliance for children and seniors with easy adherence to a proper dosage regimen, because they are chemically unstable and susceptible to hydrolysis similarly to other β-lactam compounds. Penem compounds generally have a poor stability in the presence of a compound having a proton-donating group such as sugars and organic acids, and it has been difficult to ensure stability of penem compounds in solution and thus the need for their formulation in a dry syrup has not been met

According to the present invention, it is possible to provide oral granular formulations in the form of a dry syrup which contains a penem compound such as compound 1 as an active ingredient, and yet which exhibits rapid disintegration, dissolution or suspension and stability.

According to the present invention, oral granular formulations with excellent disintegrating or dissolving properties can be provided optionally by adding further various additives with the aim of improving stability after dissolution or suspension or stability in the solid state. Thus, various additives were carefully examined with a view to ensuring the stability of the pharmaceutical composition of the present invention containing a penem compound, such as compound 1, as an active ingredient. As a result, it was found surprisingly that the stability of penem compounds both in aqueous solution and solid state can be improved by adding D-mannitol among various sugars that generally destroy the stability of compound 1. Therefore, the present invention provides stable dry syrups containing a penem compound as an'active ingredient and having fast-disintegrating or -dissolving properties.

The amount of D-mannitol also serving as an excipient and a sweetening agent in the composition of the present invention is not specifically limited, but can be appropriately selected from a range that ensures the stability of active ingredients both in water and in a solid state and imparts pharmaceutically desirable properties to the compositions. In order for the pharmaceutical composition to be water-soluble, the amount of D-mannitol is preferably 30% by weight or less of the total weight of the compositions when it is desired that formulations having an indicated potency of 10% become clear at a concentration of active ingredients of 40 mg (potency)/ mL. Compositions containing compound 1 as an active ingredient preferably contain D-mannitol in an amount of 5% by weight or more of the total weight of the compositions with regard to stability after being dissolved in water.

### EXAMPLES

The following examples further illustrate the present invention without, however, limiting the scope of the invention thereto.

### Example 1

The influence of sucrose on granulation and disintegration of oral granular formulations was evaluated. About 200 g of each of mixed powders consisting of compound 1 and sucrose in the ratios shown in Table 1 was blended in a fluidized bed granulator (New Marumerizer NQ-LABO from Fuji Paudal) for 2 minutes (product temperature: 24°C, inlet air flow: dial setting 4-5, inlet air pressure: 50-100 mmAq). The mixture was granulated for 40-60 minutes while it was sprayed with water at a rate of 1.0-1.4 g/min (product temperature: 24°C, inlet air temperature: 30-40°C, inlet air flow: dial setting 4-5, inlet air pressure: 50-100 mmAq). Then, the granules were dried in the same apparatus for 5-10 minutes (product temperature: 24-30°C, inlet air temperature: 30-40°C, inlet air flow: dial setting 4-5, inlet air pressure: 50-100 mmAq). The granules were removed and screened through a sieve No. 30 (500 µm) into 5 classes of granules.

By assessing the proportion of fine powder, completion of granulation of the resulting 5 classes of granules was evaluated based on one of the definitions for fine granules provided in the 12th edition of the Japanese Pharmacopoeia. That is, granulation was judged to be good when the proportion of fine powder passing through a sieve No. 200 (75 µm) is 10% by weight or less than the total weight. The sieve was operated according to the method described in the 12th edition of the Japanese Pharmacopoeia.

As a result, granules containing sucrose at a proportion of 30% by weight or more (samples 1-3) showed good granulation among these formulations consisting of sucrose and compound 1 (Table 1).

The three classes of granules showing good granulation were evaluated for disintegration by simulating an environment where the amount of water is not sufficient such as the inside of the mouth. That is, a ring having a diameter of 12 mm and a height of 20 mm was mounted on a filter paper impregnated with water, about 0.5 g of granules were placed in the ring and the appearance of the granules was observed after 2 minutes. As a result, all of the three classes of granules showed rapid disintegration irrespective of the ratio of sucrose (Table 1).

Thus, it was found that the pharmaceutical composition of the present invention granulated with sucrose shows good granulation without using binders. It was also found that varying amounts of sucrose do not affect rapid disintegration of oral granular formulations. For the pharmaceutical composition containing compound 1 as an active ingredient without any other ingredients, good granulation was shown when 30% by weight or more of sucrose was added.

**Table 1**

| Sample No. | | 1 | 2 | 3 | 4 | 5 |
|---|---|---|---|---|---|---|
| Composition (% by weight) | Compound 1 | 10.0 | 50.0 | 70.0 | 80.0 | 90.0 |
| | Sucrose | 90.0 | 50.0 | 30.0 | 20.0 | 10.0 |
| Evaluation of granulation* | | o | o | o | x | x |
| Evaluation of disintegration** | | o | o | o | - | - |

| | | | | | | |
|---|---|---|---|---|---|---|
| * o means that the proportion of fine powder of 75 µm or less is 10% by weight or less, and x means that the proportion of fine powder of 75 µm or less exceeds 10% by weight. | | | | | | |
| ** o means that any granular shape is not found 2 minutes after 0.5 g of each sample is placed in a ring (diameter 12 mm, height 20 mm) mounted on a filter paper impregnated with water. ** - not evaluated. | | | | | | |

### Example 2

Pharmaceutical compositions containing excipients as other additives were prepared and the influence of sucrose on granulation and disintegration was evaluated. Five classes of granules shown in Table 2 were prepared by the same procedure as in Example 1 and granulation and disintegration were evaluated.

As a result, 3 classes of granules (samples 6-8) containing sucrose showed good granulation and rapid disintegration. However, granules not containing sucrose (samples 9, 10) showed insufficient granulation (Table 2). Thus, it was found that oral granular formulations containing at least 30% by weight of sucrose show improved granulation and excellent disintegration as compared with those not containing sucrose.

**Table 2**

| Sample No. | | 6 | 7 | 8 | 9 | 10 |
|---|---|---|---|---|---|---|
| Composition (% by weight) | Compound 1 | 10.0 | 10.0 | 10.0 | 10.0 | 10.0 |
| | Sucrose | 30.0 | 30.0 | 30.0 | - | - |
| | Lactose | 60.0 | - | - | - | - |
| | Corn starch | - | 60.0 | - | 90.0 | - |
| | Microcrystalline cellulose | - | - | 60.0 | - | 90.0 |
| Evaluation of granulation* | | o | o | o | x | x |
| Evaluation of disintegration** | | o | o | o | - | - |

| | | | | | | |
|---|---|---|---|---|---|---|
| * o means that the proportion of fine powder of 75 µm or less is 10% by weight or less, and x means that the proportion of fine powder of 75 µm or less exceeds 10% by weight. | | | | | | |
| ** o means that any granular shape is not found 2 minutes after 0.5 g of each sample is placed in a ring (diameter 12 mm, height 20 mm) mounted on a filter paper impregnated with water. ** - not evaluated. | | | | | | |

### Example 3

Pharmaceutical compositions containing binders as other additives were evaluated for the influence of sucrose on granulation and disintegration. About 200 g of each of mixed powders having the compositions shown in Table 3 except for hydroxypropylcellulose was blended in a fluidized bed granulator (New Marumerizer NQ-LABO from Fuji Paudal) for 2 minutes (product temperature: 24°C, inlet air flow: dial setting 4-5, inlet air pressure: 50-100 mmAq). The mixture was granulated for about 40-120 minutes while it was sprayed with an aqueous hydroxypropylcellulose solution (sample 11) or water (sample 12) at a rate of 1.0-1.4 g/min (product temperature: 24°C, inlet air temperature: 30-40°C, inlet air flow: dial setting 4-5, inlet air pressure: 50-100 mmAq). Then, the granules were dried in the same apparatus for 5-10 minutes (product temperature: 24-30°C, inlet air temperature: 30-40°C, inlet air flow: dial setting 4-5, inlet air pressure: 50-100 mmAq). The granules were removed and screened through a sieve No. 30 (500 µm) into 2 classes of granules. The resulting granules were evaluated with regard to granulation and disintegration by the method above.

As a result, granules containing 10% by weight of a binder but not sucrose (sample 11) showed good granulation, but extremely poor disintegration. In contrast, granules in which sucrose was used in place of the binder for improving disintegration (sample 12) exhibited rapid disintegration while maintaining good granulation (Table 3). Thus, it was found that the addition of 10% by weight of sucrose can reduce the amount of binders, which are a main cause of delayed disintegration, while ensuring good granulation.

**Table 3**

| Sample No. | | 11 | 12 |
|---|---|---|---|
| Composition (% by weight) | Compound 1 | 10.0 | 10.0 |
| | Sucrose | 0.0 | 10.0 |
| | Lactose | 80.0 | 80.0 |
| | Hydroxypropylcellulose | 10.0 | 0.0 |
| Evaluation of granulation* | | o | o |
| Evaluation of disintegration** | | x | o |

| | | | |
|---|---|---|---|
| * o means that the proportion of fine powder of 75 µm or less is 10% by weight or less, and x means that the proportion of fine powder of 75 µm or less exceeds 10% by weight. | | | |
| ** o means that any granular shape is not found 2 minutes after 0.5 g of each sample is placed in a ring (diameter 12 mm, height 20 mm) mounted on a filter paper impregnated with water. | | | |

### Example 4

Oral granular formulations having the compositions shown in Table 4 were prepared. Namely, compound 1, sucrose and D-mannitol were mixed in a fluidized bed granulator (STREA-I from Powrex) for about 15 minutes (outlet air temperature: 30-34°C, inlet air flow: 20 m³/hr). The mixture was granulated for about 30 minutes while it was sprayed with water at a rate of 3.0-4.0 g/min (outlet air temperature: 31-32°C, inlet air flow: 10-40 m³/hr). Then, the granules were dried in the same apparatus for about 30 minutes (outlet air temperature: 34-37°C, inlet air flow: 20 m³/hr). The granules were removed and screened through a sieve No. 30 (500 µm) into 2 classes of granules. A glass bottle containing 3 g of the resulting granules was tightly sealed and stored at 60°C for 12 days to determine the content of compound 1.

The content of compound 1 in the samples was determined by high-performance liquid chromatography as follows. A stainless steel high-performance liquid chromatography column packed with octadecylsilyl silica gel was used. The column temperature was set at 40°C. The mobile phase consisted of a mixed solution of 840 mL of a solution containing 45 mM potassium dihydrogenphosphate, 5 mM sodium monohydrogenphosphate and 5 mM tetra-n-butylammonium bromide and 160 mL of acetonitrile. The flow rate was controlled to give the retention time of compound 1 of 11 minutes. For detection, a UV absorption spectrophotometer was used at a wavelength of 305 nm.

As a result, granules not containing D-mannitol (sample 13) showed a yellowish appearance after storage and the residual retention to the initial potency of compound 1 was 83.0%. However, granules containing 5% by weight of D-mannitol (sample 14) was less colored than sample 13 and the residual retention to the initial potency was 91.1%, i.e., higher than that of sample 13 (Table 4). This showed that the addition of at least 5% by weight of D-mannitol in the solid state has the effect of improving the stability of compound 1 in oral granular formulations of the present invention.

**Table 4**

| Sample No. | | 13 | 14 |
|---|---|---|---|
| Composition (mg) | Compound 1 | 123.5 | 123.5 |
| | Sucrose | 876.5 | 826.5 |
| | D-mannitol | 0 | 50 |
| Stability test results | Initial appearance | White granules | White granules |
| | Appearance after storage at 60°C/12 days | Yellow granules (aggregated) | Pale yellow granules (aggregated) |
| | Potency retention (%) after storage at 60°C/12 days | 83.0 | 91.1 |

### Example 5

The influence of D-mannitol on the stability of compound 1 in aqueous solution containing sucrose was evaluated. An aqueous solution of each of the mixed powders having the formulations shown in Table 5 was prepared at a concentration of compound 1 of 49.4 mg/mL (40.0 mg (potency)/mL). A glass bottle containing 5 mL of the resulting aqueous solution was tightly sealed and stored at 25°C for 7 days to determine the content of compound 1. The content of compound 1 was determined by the HPLC method above.

As a result, the residual retention to the initial potency in a sample not containing D-mannitol (sample 15) after storage at 25°C for 7 days was 83.5%. However, the residual retentions to the initial potencies in all the samples containing 4.9 - 29% by weight of D-mannitol (samples 16-19) were about 90% (Table 5). This showed that D-mannitol has the effect of improving the stability of compound 1 in aqueous solution containing sucrose. Thus, it was found that pharmaceutical compositions of the present invention containing compound 1, a water-labile penem compound, as an active ingredient can be made sufficiently stable in a solution or dispersion in water by incorporating about 5% by weight or more of D-mannitol based on the weight of the compositions.

**Table 5**

| Sample No. | | 15 | 16 | 17 | 18 | 19 |
|---|---|---|---|---|---|---|
| Composition (mg/ml) | Compound 1 | 49.4 | 49.4 | 49.4 | 49.4 | 49.4 |
| | Sucrose | 360 | 340 | 320 | 280 | 240 |
| | D-mannitol | 0 | 20 | 40 | 80 | 120 |
| Stability test results | Potency retention (%) after storage at 25°C/7 days | 83.5 | 89.3 | 91.2 | 91.6 | 92.0 |

### Example 6

The following test was performed for the purpose of obtaining an approximate measure of the upper limit of the amount of D-mannitol to be added to oral granular formulations of the present invention of the type used as pharmaceutical compositions for which it is required to be soluble in water.

Each of mixed powders having the formulations shown in Table 6 was dissolved or suspended in water at a concentration of compound 1 of 49.4 mg/ml (40.0 mg (potency)/mL) to evaluate the appearance (liquid appearance) of the aqueous solution or suspension. As a result, a formulation containing 29.3% by weight of D-mannitol based on the weight of the composition (sample 20) was completely dissolved to become clear. However, formulations containing 34.2% by weight (sample 21) and 39.1% by weight (sample 22) of D-mannitol were found to leave an insoluble ingredient (Table 6). This insoluble ingredient was determined to be D-mannitol as was also indicated by the documentary saturation solubility.

Thus, oral granular formulations of the present invention should desirably contain D-mannitol in an amount of about 30% by weight or less based on the weight of the compositions when they are pharmaceutical compositions which are to be soluble in water. However, the amount of D-mannitol contained in oral granular formulations of the present invention can be appropriately increased when they are dissolved in a larger amount of water than indicated above or when they are acceptable as a suspension.

**Table 6**

| Sample No. | | 20 | 21 | 22 |
|---|---|---|---|---|
| Composition (mg/ml) | Compound 1 | 49.4 | 49.4 | 49.4 |
| | Sucrose | 240 | 220 | 200 |
| | D-mannitol | 120 | 140 | 160 |
| Liquid appearance | | Clear | Suspension | Suspension |

### Example 7: Preparation of dry syrups

### [Preparation examples 1-3 of dry syrup]

Dry syrups having the compositions shown in Table 7 were prepared by the fluidized bed granulation process. Namely, compound 1, sucrose. D-mannitol and magnesium aluminometasilicate (for sample 24) were mixed in a fluidized bed granulator (STREA-I from Powrex) for about 15 minutes (outlet air temperature: 30-34°C, inlet air flow: 20 m³/hr). The mixture was granulated for about 30 minutes while it was sprayed with an aqueous solution separately prepared by dissolving hydroxypropylcellulose (for samples 23 and 24) and banana flavor in water at a rate of 3.0-4.0 g/min (outlet air temperature: 31-32°C, inlet air flow: 10-40 m³/hr). After completion of granulation, the granules were dried in the same apparatus for about 30 minutes (outlet air temperature: 34-37°C, inlet air flow: 20 m³/hr). The granules were removed and screened through a sieve No. 30 (500 µm) into 3 white dry syrups. The resulting dry syrups were subjected to pharmaceutical evaluations.

Particle size distribution analysis of each dry syrup showed that the proportion of fine powder of 75 µm or less was 10% by weight or less in all the samples, verifying good granulation of the dry syrup irrespective of the presence or absence of binders. All the dry syrups also showed rapid disintegration as evaluated by the method above. When purified water was added to these dry syrups at a concentration of compound 1 of 49.4 mg/mL (40.0 mg (potency)/mL), all of them rapidly disintegrated so that the dry syrup containing an insoluble matter magnesium aluminometasilicate (sample 24) formed a white suspension while the other two dry syrups (samples 23 and 25) formed colorless clear aqueous solutions (Table 7).

Thus, it was found that pharmaceutical compositions of the present invention in the form of a dry syrup show good granulation and rapidly disintegrate into solution or suspension. It was also found that dry syrups which are homogeneously soluble in water can be provided by appropriate selections of the composition of additives.

**Table 7**

| Sample No. | | 23 | 24 | 25 |
|---|---|---|---|---|
| Composition (% by weight) | Compound 1 | 12.35 | 12.35 | 12.35 |
| | Sucrose | 55.85 | 55.85 | 57.85 |
| | D-mannitol | 29.80 | 29.30 | 29.80 |
| | Hydroxypropylcellulose | 2.00 | 2.00 | 0.00 |
| | Mg aluminometasilicate | 0.00 | 0.50 | 0.00 |
| | Banana flavor | trace | trace | trace |
| Appearance | | White granules | White granules | White granules |

| Evaluation of granulation by particle size distribution (%) | | | | |
|---|---|---|---|---|
| | 500 µm or more | 0.2 | 0.1 | 0.1 |
| | 355-500 µm | 13.7 | 8.4 | 5.7 |
| | 177-355 µm | 75.2 | 81.9 | 71.2 |
| | 75-177 µm | 8.6 | 7.1 | 19.6 |
| | 75 µm or less | 2.3 | 2.5 | 3.4 |
| Evaluation of disintegration* | | o | o | o |
| Liquid appearance** | | Colorless clear | White suspension | Colorless clear |

| | | | | |
|---|---|---|---|---|
| * o means that any granular shape is not found 2 minutes after 0.5 g of each sample is placed in a ring (diameter 12 mm, height 20 mm) mounted on a filter paper impregnated with water. | | | | |
| ** Liquid appearance was observed when 4 g of each sample was dissolved or dispersed in 10 ml of water. | | | | |

### [Preparation example 4 of dry syrup]

A dry syrup having the composition shown in Table 8 was prepared by the fluidized bed granulation process. Namely, about 4.9 kg of a mixed powder consisting of compound 1, sucrose and D-mannitol was blended in a fluidized bed granulator (New Marumerizer NQ-230 from Fuji Paudal) for 2 minutes. The mixture was granulated for about 50 minutes while it was sprayed with a binder solution separately prepared by dissolving hydroxypropylcellulose and colorant Yellow No. 5 in water. Subsequently, the mixture was sprayed with a flavoring solution of orange flavor diluted in ethanol for about 10 minutes. Then, the granules were dried in the same apparatus for about 10 minutes. The granules were removed and screened through a sieve No. 30 (500 µm) into an orange dry syrup. The resulting dry syrup was subjected to pharmaceutical evaluations.

Particle size distribution analysis of this dry syrup showed that the proportion of fine powder of 75 µm or less was 5.4% by weight, verifying good granulation of this composition. This dry syrup also showed rapid disintegration as evaluated by the method above. When purified water was added at a concentration of compound 1 of 49.4 mg/mL (40.0 mg (potency)/mL), this dry syrup rapidly disintegrated and rapidly dissolved into a clear orange solution (Table 8).

The stability after dissolved in water was also evaluated. A glass bottle containing 5 mL of an aqueous solution of this dry syrup dissolved in water at a concentration of compound 1 of 49.4 mg/mL (40.0 mg (potency)/mL) was tightly sealed and stored at 10°C for 28 days to determine the appearance of the liquid and the content of compound 1. The content of compound 1 in the composition was determined by the method above.

As a result, no change was observed in liquid appearance before and after storage at 10°C for 28 days. The residual retention to the initial potency after storage was 94.2% (Table 8). Thus, this dry syrup containing D-mannitol showed good stability after dissolved in water.

**Table 8**

| Sample No. | | 26 |
|---|---|---|
| Composition | Compound 1 | 12.35 |
| **(%** by weight) | Sucrose | 55.85 |
| | D-mannitol | 29.75 |
| | Hydroxypropylcellulose | 2.00 |
| | Colorant Yellow No. 5 | 0.05 |
| | Orange flavor | **trace** |
| Appearance | | Orange granules |
| Odor | | Orange-like |

| Evaluation of granulation by particle size distribution (%) | | |
|---|---|---|
| | 500 µm or more | 0.2 |
| | 355-500 µm | 11.3 |
| | 177-355 µm | 72.5 |
| | 75-177 µm | 10.6 |
| | 75 µm or less | 5.4 |
| Evaluation of disintegration* | | o |
| Liquid appearance** | | Clear orange |

| Stability test results after dissolution | | |
|---|---|---|
| | Initial liquid appearance | Clear orange |
| | Liquid appearance after 10°C/28 days | Clear orange |
| | Potency retention (%) after 10°C/28 days | 94.2 |

| | | |
|---|---|---|
| * o means that any granular shape is not found 2 minutes after 0.5 g of each sample is placed in a ring (diameter 12 mm, height 20 mm) mounted on a filter paper impregnated with water. | | |
| ** Liquid appearance was observed when 4 g of each sample was dissolved or dispersed in 10 ml of water. | | |

Thus, it was confirmed that pharmaceutical compositions of the present invention provide dry syrups containing compound 1, a penem compound, as an active ingredient, which have good granulation and rapid disintegration, homogeneously dissolve in water into clear solution and are stable after dissolved in water.

## Claims

1. A fast-disintegrating porous granular oral pharmaceutical composition in a dosage form to be dissolved or suspended before use, wherein said composition is prepared by fluidized bed granulation with sucrose and contains a penem compound as an active ingredient and not less than 30% by weight of sucrose based on the total weight of the composition.

2. The oral pharmaceutical composition of claim 1, wherein the penem compound is faropenem sodium.

3. The oral pharmaceutical composition of claim 2, which is in the form of a dry syrup.

4. The oral pharmaceutical composition of claim 3, which provides a clear solution when dissolved in water.

5. The oral pharmaceutical composition of any one of claims 1-4, which contains D-mannitol.

6. The oral pharmaceutical composition of claim 5 containing from 5 to 30% by weight of D-mannitol based on the total weight of the composition.

## Patentansprüche

1. Sich schnell auflösende poröse granulierte orale pharmazeutische Zusammensetzung in einer Dosierungsform, die vor der Verwendung aufgelöst oder suspendiert wird, wobei die Zusammensetzung hergestellt wird durch Fließbettgranulierung mit Sucrose und eine Penemverbindung als aktiven Bestandteil und nicht weniger als 30 Gew.% Sucrose auf Basis des Gesamtgewichts der Zusammensetzung enthält.

2. Orale pharmazeutische Zusammensetzung nach Anspruch 1, wobei die Penemverbindung Faropenemnatrium ist.

3. Orale pharmazeutische Zusammensetzung nach Anspruch 2, die in Form eines trockenen Sirups vorliegt.

4. Orale pharmazeutische Zusammensetzung nach Anspruch 3, die, wenn in Wasser aufgelöst, in einer klaren Lösung vorliegt.

5. Orale pharmazeutische Zusammensetzung nach einem der Ansprüche 1 bis 4, die D-Mannitol enthält.

6. Orale pharmazeutische Zusammensetzung nach Anspruch 5, die 5 bis 30 Gew.% D-Mannitol auf Basis des Gesamtgewichts der Zusammensetzung enthält.

## Revendications

1. Composition pharmaceutique pour administration orale à base de granulés poreux à décomposition rapide sous une forme posologique à dissolution ou à suspension avant emploi, dans laquelle ladite composition est préparée par granulation à lit fluidisé avec du saccharose et contient un composé de pénème en tant qu'ingrédient actif et pas moins de 30 % en poids de saccharose en se basant sur le poids total de la composition.

2. Composition pharmaceutique pour administration orale selon la revendication 1, dans laquelle le composé de pénème est du sodium de faropénème.

3. Composition pharmaceutique pour administration orale selon la revendication 2, qui est sous forme de sirop à l'état sec.

4. Composition pharmaceutique pour administration orale selon la revendication 3, donnant une solution claire lorsqu'elle est dissoute dans l'eau.

5. Composition pharmaceutique pour administration orale selon l'une quelconque des revendications 1 à 4, contenant du D-mannitol.

6. Composition pharmaceutique pour administration orale selon la revendication 5, contenant 5 à 30 % en poids de D-mannitol en se basant sur le poids total de la composition.
